# EUROPEAN PATENT SPECIFICATION

(11) **EP 2 674 172 B1**
(45) Date of publication and mention of the grant of the patent: **15.02.2017**
(21) Application number: 12171457.0
(22) Date of filing: 11.06.2012
(51) Int. Cl.: A61K 9/00, A61K 48/00

(54) **Pharmaceutical formulation for ultrasound transfection**
Pharmazeutische Formulierung für Ultraschalltransfektion
Formulation pharmaceutique pour transfection à ultrasons

(43) Date of publication of application: 18.12.2013
(73) Proprietor: Ludwig Boltzmann Gesellschaft, 1200 Wien (AT)
(72) Inventor: Feichtinger, Georg, 1060 Wien (AT); Mc Hale, Anthony P., Coleraine, BT52 1SA (GB); Nomikou, Nikolitsa, Dublin 5 (IE); Redl, Heinz, 1060 Wien (AT)
(74) Representative: Sonn & Partner Patentanwälte

(56) References cited:
- US-A1- 2001 031 740
- X. WANG ET AL: "Gene Transfer with Microbubble Ultrasound and Plasmid DNA into Skeletal Muscle of Mice: Comparison between Commercially Available Microbubble Contrast Agents", RADIOLOGY, vol. 237, no. 1, 1 January 2005 (2005-01-01), pages 224-229, XP055047809, ISSN: 0033-8419, DOI: 10.1148/radiol.2371040805
- CHRISTIANSEN J P ET AL: "Targeted tissue transfection with ultrasound destruction of plasmid-bearing cationic microbubbles", ULTRASOUND IN MEDICINE AND BIOLOGY, NEW YORK, NY, US, vol. 29, no. 12, 1 December 2003 (2003-12-01), pages 1759-1767, XP004482543, ISSN: 0301-5629, DOI: 10.1016/S0301-5629(03)00976-1

## Description

The present invention relates to compositions and methods for use in tissue or organ regeneration, especially in connection with damage of bone.

In the treatment of tissue or organ defects, particularly in the case of severe trauma relating to damage of bone, surgical procedures are required to repair defects. In many cases, the trauma may be so severe that removal of significant quantities of tissue is necessitated and in cases such as severe limb trauma, this may result in limb shortening or fracture non-union. The above may also apply to removal of tissues during the surgical treatment of cancer and such treatment may, again, lead to severe impairment or complete absence of regeneration requiring a true functional replacement. Bone grafting and distraction osteogenesis are currently clinically applied to achieve regeneration. These procedures are invasive, cumbersome and associated with increased morbidity of the patient. The emerging field of tissue engineering appears to provide a promising resolution for such cases, where, hypothetically, removed or damaged tissues may be replaced using autologous stem cell populations derived from the patient in order to regenerate the original damaged tissues.

In terms of existing practical applications of stimulating the regeneration of bone tissues, it is now relatively routine to employ morphogenic factors such as bone morphogenetic proteins (BMP) in the clinic and autologous mesenchymal stem cells are considered as promising therapeutics to support bone formation at the defect site.

Cell and growth factor delivery for tissue regeneration requires the use of biocompatible scaffolds/matrices to provide a structural basis for the newly formed tissue as well as to immobilize the delivered components in place at the defect site. Furthermore, depending on the applied material, these matrices can itself support cellular differentiation, proliferation, matrix deposition, growth factor bioactivity and release profiles.

As an alternative to direct to growth factor application to stimulate cellular differentiation, several local gene therapeutic approaches including the matrix based delivery of transfected cells that express a therapeutic growth factor or the use of so called gene activated matrices to transfect cells in vivo within the matrices have been investigated with varying outcome. This emerging field of gene therapy based tissue engineering provides a promising alternative to the above described classical tissue engineering approach with superior safety profiles (if the gene is delivered non-virally), at lower cost and with potentially superior outcome compared to growth factor based approaches.

If stem cells are immobilised in a matrix in order to facilitate the regeneration of tissues and if the morphogenesis of those cells is to be controlled by expression of exogenous genes, then a convenient, non-invasive means of facilitating gene transfer or transfection would be required. Of the various methods available to facilitate transfection for the purpose of expressing exogenous and/or endogenous genes for therapeutic reasons, these may be divided into two major categories, namely, viral and non-viral procedures. Although the majority of protocols currently employ viruses for clinical trials, a number of challenges remain. These include the random insertion of the viral nucleic acid into the genome of the recipient host/patient with negative consequences and the elicitation of adverse immune responses that subsequently preclude repeat challenge with the viral-based gene therapeutics.

In terms of non-viral therapies the gene transfection modalities are many and varied and include the use of cationic carriers, ballistic methods, use of Trojan peptide-based systems and electroporation and sonoporation-based system. Of these, electroporation has received most clinical attention because it is compatible with delivery of nucleic acid-based medicines to topical locations and/or to hollow organs. Nevertheless, this approach does necessitate direct electrical contact with the target site and involves some degree of invasion if that target site is internal. More recently, sonoporation, or the use of ultrasound to induce transient cell membrane permeabilisation has emerged as a promising physical alternative to electroporation, since it does not involve the use of leads and electrodes configurations to enable direct electrical contact with the relevant target and so, is truly recognised as a non-invasive stimulus of transfection.

US 2001/031740 A1 discloses methods for delivering compounds comprising an organic halide into a cell. Wang et al. (Radiology 237 (1) (2005): 224-229) report gene transfer with microbubble ultrasound and plasmid DNA into skeletal muscle of mice, specifically a comparison between commercially available microbubble contrast agents (Optison, SonoVue and Levovist). Christiansen et al. (Ultrasound in Med. Biol. 29 (12) (2003): 1759-1767) describe targeted tissue transfection with ultrasound destruction of plasmid-bearing cationic microbubbles.

It is an object of the present invention to improve the current means and methods for matrix-based gene transfection. Specifically, improvement of ultrasound based therapies (sonoporation) is an object of this invention, which allows suitable gene transfection for tissue regeneration following surgery.

Therefore, the present invention provides a pharmaceutical composition comprising
- a soluble matrix precursor , wherein the soluble matrix precursor is an alginate, collagen, hyaluronic acid, fibrinogen, gelatin, chondroitin sulfate or combinations thereof,
- an ultrasound transfection agent, and
- a pharmaceutically active nucleic acid, and
- target cells for the pharmaceutically active nucleic acid, wherein the target cells are primary cells, expanded cells or a stromal cell fraction, especially fibroblasts, bone progenitor cells or adult mesenchymal stem cells.

The soluble matrix precursor can be applied to a patient and processed to become a matrix in the patient wherein the ultrasound transfection agent and the pharmaceutically active nucleic acid are dispersed in the matrix that contains the cells, especially patient cells. Then ultrasound may be applied (via the ultrasound transfection agent) in order to transform cells in or around the matrix in the patient with the pharmaceutically active nucleic acid. Alternatively, instead of a soluble matrix precursor, the matrix can be made before implantation in the patient and implanted as a matrix.

As mentioned above, sonoporation employs ultrasound to facilitate transient cell membrane permeabilisation and it has been shown to be highly effective in facilitating entry of nucleic acid into cells to enable transfection and subsequent expression of a transgene. In recent years, the number of reports describing ultrasound-mediated gene transfer has increased dramatically indicating the degree to which the field is beginning to accept this technology as a realistic alternative to existing gene transfer technologies. With the present invention a combination of different methodological set-ups (gene activated matrices, sonoporation, inducible gene switches, etc.) is shown to result in a viable and practical method which makes use of the advantageous properties of each method but at the same time avoiding the disadvantages of each of the methods applied: Whereas gene activated matrices are usually reported to have very low transfection efficiency and an active cell membrane permeabilisation cannot take place, sonoporation is usually limited by the availability of gene transfer vehicles at the proximity of the transfectable tissues or cells within the defect site. With the present invention it was not only shown that all these methods are combinable in principle, but also that the functionality of the system according to the present invention safeguards a (prolonged) targeted delivery of the nucleic acid exactly to the intended site of action thereby increasing both, the transfection efficiency and the economic feasibility of the gene transfer in total. This leads to highly increased transfection efficiency and an enhanced yield of transfected cells at the administration site which allows - due to the permanent vicinity of cells, pharmaceutically active nucleic acid and ultrasound transfection agents - tissue and bone repair with a hitherto unprecedented efficiency. Furthermore, off-target effects can be reduced since all of the components that are required for efficient therapeutic gene transfer are confined to the defect site through incorporation into applied hydrogel matrix, thus further enhancing safety of this tissue regenerative gene therapy approach. However, ultrasound gene transfer in three-dimensional matrices was not possible until the present invention enabled e.g. sonoporation of fibrin/collagen hydrogels. Accordingly, sonoporation as e.g. described in US 5,270,300 A or US 5,763,416 A was not possible to apply to gene transfer in three-dimensional matrices.

Because of its non-invasive nature, ultrasound can be employed to facilitate gene transfection in a multiplicity of targets in a multiplicity of environments and so it is highly compatible with facilitating gene transfection in cells in culture (suspension/adherent), cells in tissues (in vivo) and by implication, cells immobilised in matrices. According to the present invention, ultrasound provides an ideal means of facilitating gene transfer to stem cells entrapped within or host cell homing into a tissue regeneration matrix in order to extracorporeally control morphogenesis and maturation of those cells. Indeed the approach using ultrasound can also be employed to facilitate gene transfer in a matrix, embedded deep within tissues in a post-surgical scenario where gene transfection may be essential at some time point post-surgical implantation of the matrix entrapped stem cells.

As mentioned above, there are two major embodiments of the present invention: the first employs an easy-to-apply liquid composition, especially a liquid hydrogel basis (i.e. a liquid gelling agent which, upon contact with water forms a (hydro(gel)) as the soluble matrix precursor. Such a liquid formulation can be brought to a suitable location in the patient, e.g. a cavity, by various means for administration of liquid pharmaceutical compositions to a patient. For example, the composition can be non-invasively injected and/or surgically moulded into a cavity (e.g. for non-union fracture (pseudoarthrosis, critical size defect; a fracture which does not heal, and leads to generation of fibrotic scar tissue in the fracture area, regeneration after serious trauma)) where it is allowed to set, for example by adding a matrix activator which allows the soluble matrix precursor to become a matrix (e.g. by adding a polymerisation inducer, adding a cleaving agent (e.g. fibrinogen is contacted with a thrombin activity) or adding Ca²⁺ ions (to alginate)). Other matrix precursors can be transformed to the matrix by applying physical measures (e.g. cooling (e.g. in the case of gelatine)). Once the matrix according to the present invention is established in the intended place in the patient by surgery, the wound can be closed and permitted to heal. Following a sufficient time period post introduction depending on component stability, ultrasound can be applied to the skin or tissue surface overlying the matrix and ultrasound-mediated transfection is accomplished. The ultrasound application can, in principle, be applied soon after the introduction (post operationem; e.g. 1 or 2 hours after the introduction (or even immediately thereafter)). However, it is preferred to apply the ultrasound a few days or even a few weeks after implantation. Preferably, the wound is allowed to heal and ultrasound is applied thereafter. For example, ultrasound may be applied 3, 4, 5, 6 or 7 days after introduction or, alternatively after 1 week, 10 days or 2 weeks. Since all relevant components are present at the intended position for action, application of ultrasound can be applied repeatedly, for example 2, 3, 4, 5, 6, 7, 8, 9 or 10 times or even more frequent, depending e.g. on the development of the bone/tissue repair process in the individual patient. Repetitive sonoporation is specifically suited in the present invention because of the immobilisation of the components in the matrix at the site of action. In the case that cells of the patients should be allowed to move into the matrix established in the patient according to the present invention, sonoporation could be conducted preferably after 1 week, 10 days or even 2 weeks after implantation to allow a sufficient number of the patient's cells to move into the matrix. If the cells are already applied with the matrix or the matrix precursor, sonoporation may be performed more quickly after introduction at the relevant site in the patient.

A "soluble matrix precursor component" as understood in the present invention is an agent that is initially soluble and subsequently converted into an insoluble matrix by other soluble precursor components. For example, fibrinogen can be a preferred soluble matrix precursor component according to the present invention. Fibrinogen is soluble and can be converted into fibrin, an insoluble matrix by thrombin (another soluble matrix precursor component). Thrombin can also act as the matrix activator (Ferguson et al., "Fibrin: The Very First Biomimetic Glue - Still a Great Tool", p.226; Nürnberger et al., "Properties and Potential Alternative Applications of Fibrin Glue", p.240; both in: J. von Byern and I. Grunwald, eds.; "Biological Adhesive Systems: From Nature to Technical and Medical Application" (2010), Springer). Another preferred example for a soluble matrix precursor component may be collagen at one pH which can be converted into a gel matrix if the pH is adjusted (collagen is soluble in hydrochloric acid; a collagen solution can be provided at pH 3.0 and when it is neutralized using e.g. a phosphate buffer pH 11.0, the collagen can form a soft gel). In this case, the adjustment of the pH acts as the matrix activator.

Preferred soluble matrix precursors according to the present invention are gelling agents which, upon contact with liquid components, such as water or aqueous solutions (e.g. buffers), form a gel.

The pharmaceutical composition according to the present invention preferably contains a biocompatible and biodegradable material with a low or absent immunogenic property, hydrogels such as alginate, collagen, hyaluronic acid, fibrinogen, fibrin, gelatin, chondroitin sulfate, or combinations thereof as soluble matrix precursor.

Depending on the soluble matrix precursor of the pharmaceutical composition according to the present invention, the matrix precursor may be converted into a suitable matrix (e.g. after administration of the pharmaceutical composition according to the present invention) by a variety of possibilities. For example, a suitable matrix activator may be employed according to the present invention to transform the matrix precursor into the matrix (i.e. to obtain a three-dimensional scaffold from the liquid precursor). A "matrix activator" according to the present invention is a component that actively converts a soluble precursor into a gelled matrix. Examples well known in the art include thrombin that can convert soluble fibrinogen in to a fibrin matrix (Ferguson et al., 2012; Nürnberger et al., 2010). In another embodiment, the matrix activator may be a cross-linker such as a divalent cation such as Ca²⁺ or Ba²⁺, where their inclusion into solutions of a polyanionic polymer (e.g. alginate) results in crosslinking of the polymer chains and conversion to a gel (Torre et al., 2009). In a further embodiment a cross-linker, such as genipin, may also be used as a matrix activator to reinforce gels formed by the matrix activator thrombin (Schek et al., 2011). This matrix activator can be added to the matrix precursor immediately before administration of the matrix precursor; or even at an earlier stage (if the process for transforming the precursor into the final matrix is slow enough or controllable by other means (e.g. temperature, ion composition, etc.); or at a later stage (at a point in time when the matrix precursor has already been administered to the patient). Examples of suitable matrix activators are polymerisers, such as acrylate for hyaluronic acid; Ca²⁺ for alginate; thrombin for fibrinogen.

Other measures for converting matrix precursors into the matrix (as a set of conditions or combinations of converting conditions and provision of matrix precursors) include the use of physical means, such as temperature changes, ultrasound, laser, other electromagnetic radiation (UV, photopolymerisation), etc.. For example, gelatine can be applied by warming the gelatine until it is in the liquid form, introduction of the gelatine to the relevant site in the patient (at least at this stage also the components, such as the nucleic acid or the ultrasound transfection agent are introduced, if they had not been admixed to the gelatine before), and allowing to solidify at the site of action. The measure for converting the matrix precursor to the matrix may also be a change in pH. One example of this is the provision of soluble collagen solutions by commercial providers (e.g. Devro, UK, EPC Inc., USA, Collagen Solutions LLC, USA) at low pH (e.g. pH 3.0) and subsequent adjustment to neutral pH (pH 7.0) results on conversion to a gel. Alternatively, the gelatine can also be solidified (e.g. in an appropriate form and already admixed with the other components) externally and the solidified matrix may then be introduced in the patient. For alginate, an aqueous alginate solution can be admixed with a Ca²⁺-containing solution (e.g. Ca-citrate) thereby forming the gel (again, at the site of action or before introduction whereafter the solidified matrix is introduced into the patient. Alternatively, the conversion from matrix precursor to matrix can also be performed as simple gelling step (e.g. after administration with the use of body fluids, such as blood, present at the site of administration or externally applied liquid formulations (as "matrix activator") for building up a gel (i.e. a three-dimensional structure with a liquid phase)).

As already outlined, the matrix activator may be combined with specific process steps enabling the conversion to a matrix. For example, combination of cross-linking agents (as matrix activator) and temperature (as matrix-generating or matrix-aiding process step) can be used for matrix conversion of collagen: genipin can be used as a crosslinker for soluble collagen, the gelation of which is temperature dependant (Macaya et al., 2011; Palmer et al., 2011).

The components used for the preparation of the matrices or matrix precursors according to the present invention may be provided in a kit form, such as a multicomponent system. For example, a kit may be provided comprising the matrix substrate, optionally a crosslinker for the matrix substrate (if the matrix is e.g. solidified by crosslinking), the nucleic acid, the ultrasound transfection agent and, the cells or other components. In applying the kit components according to the present invention, administration systems for two- or three- component systems already established in surgery may preferably be applied, such as the systems used for administering fibrinogen based tissue adhesives.

If a matrix activator is used, the composition according to the present invention is preferably provided in a set form comprising at least the following components a) and b):
a) a soluble matrix precursor component, the soluble matrix precursor being an alginate, collagen, hyaluronic acid, fibrinogen, gelatin, chondroitin sulfate or combinations thereof,
b) a matrix activator component, and
c) target cells for the pharmaceutically active nucleic acid, wherein the target cells are primary cells, expanded cells or a stromal cell fraction, especially fibroblasts, bone progenitor cells or adult mesenchymal stem cells,
wherein the ultrasound transfection agent and the pharmaceutically active nucleic acid are contained either in a) or in b) or in both, preferably in a). This allows a uniform dispersion of the ultrasound transfection agent and the pharmaceutically active nucleic acid in the final matrix so that the transfection effects of sonoporation can be used most efficiently.

As already mentioned, the pharmaceutical composition according to the present invention can also be applied as a ready-to-use matrix. The matrix is made outside the patient and can be pre-shaped, if necessary, for the intended use. Accordingly, the present invention also relates to a pharmaceutical composition comprising
- a matrix , wherein the matrix is solidified alginate, solidified collagen, solidified hyaluronic acid, fibrin, solidified gelatin, solidified chondroitin sulfate or mixtures thereof,
- an ultrasound transfection agent,
- a pharmaceutically active nucleic acid and
- target cells for the pharmaceutically active nucleic acid, wherein the target cells are primary cells, expanded cells or a stromal cell fraction, especially fibroblasts, bone progenitor cells or adult mesenchymal stem cells.

This matrix is already provided in the form to be directly administered without the need to establish a three-dimensional structure in the patient and already contains the ultrasound transfection agent and the pharmaceutically active nucleic acid (as well as other ingredients needed). The matrix is preferably pre-manufactured with the components uniformly attached thereto or included (dispersed) therein.

The matrix of this pharmaceutical composition is therefore chosen from the group solidified alginate, solidified collagen, solidified, especially polymerised hyaluronic acid, fibrin, solidified gelatin, solidified fibroin, solidified, especially crosslinked chondroitin sulfate or mixtures thereof.

One particular embodiment of the current invention which is specifically preferred involves mixing a polymer together with a matrix precursor to achieve efficient ultrasound-mediated, microbubble-enhanced gene transfer. One specific example of this involves the inclusion of collagen type I into fibrinogen as a matrix precursor. The matrix activator is subsequently added to the mixture (the soluble matrix precursor) to effect matrix gel formation and the overall matrix has collagen interspersed in the resulting fibrin matrix.

Preferably, the ratio of collagen to fibrinogen in the soluble matrix precursor is from 0.01 to 0.15 % (w/v) collagen to 0.5 to 5 % (w/v) of fibrinogen, preferably from 0.03 to 0.08 % (w/v) collagen to 1 to 3 % (w/v) of fibrinogen, especially from 0.03 to 0.05 % (w/v) collagen to 1.5 to 2.5 % (w/v) of fibrinogen.

An "ultrasound transfection agent" is an agent that enhances ultrasound-mediated gene transfer into a cell. Agents that are commonly used are microbubbles comprising a shell that encloses a gas.

The ultrasound transfection agent according to the present invention therefore is an agent which enhances the effect of the ultrasound signal applied to the extent needed for transfection of the cells to be targeted by transfection with the pharmaceutically active nucleic acid (see e.g. Newman et al.; Li et al.). In the present field, the use of microbubbles, nanobubbles or mixtures thereof (the size of the bubbles usually cannot be absolutely controlled (therefore, these terms are indicative of a size range rather than an exact size); currently the use of microbubbles is widely established and therefore specifically preferred) is currently the most preferred ultrasound transfection agent. Such agents are reported to effect pores in the cell membrane by ultrasound mediated cavitation.

Microbubbles are well known in the art, for example as ultrasound contrast agents. Their composition and methods for their preparation are thus well known to those skilled in the art. Examples of procedures for the preparation of microbubbles are described in, for example, Christiansen et al., Ultrasound Med. Biol. 29 (2003), 1759 1767; Farook et al., J. R. Soc. Interface 6 (2009), 271-277; and Stride et al., Med. Biol. Eng. Comput. 47 (2009), 883-892.

Nanobubbles are the same as microbubbles except that they have diameters that are measured in nanometers and normally lower than 200 nm. In addition, nanobubbles, which may also be referred to as nanodroplets can be comprised of a liquid core that is prepared as a nanoemulsion and is subsequently converted to a gas upon exposure to ultrasound as described in, for example, Rapoport et al., Bubble Sci. Eng. Technol. 1 (2009), 31-39. A specifically preferred ultrasound transfection agent comprises a nanodroplet in which a lipid and/or polymer layer encloses an ultrasound vaporisable liquid, preferably dodecafluoropentane.

Accordingly, suitable micro- or nanobubbles in accordance with the present invention include: free gas bubbles, stabilized gas bubbles, colloidal suspensions, emulsions, and aqueous solutions. The aqueous solutions include aqueous solutions of air-filled proteinaceous microbubbles. Currently available products include gas filled liposomes, gas filled lipid bilayers, microbubbles containing liquids, gas emulsions, gas-filled microspheres and microbubbles containing suspensions (see e.g. US 5,007,438 A). Specifically preferred ultrasound transfection agents comprise a lipid monolayer enclosing a gas, preferably a perfluorocarbon gas (see e.g. US2001/031740 A1; Li et al.).

Preferred ultrasound transfection agents as enhancers of ultrasound-mediated gene transfer are microbubble preparations that are currently employed for medical imaging, such as Optison and/or SonoVue. Alternatively, microbubbles such as MB101 and SDM range (supplied commercially by Sonidel Ltd.) specifically designed for surface modification are employed and the advantage associated with the latter is to target the microbubbles to target stem cells within the microenvironment of the matrix. Such targeting includes the attachment of receptors and/or antibodies to microbubble surfaces that are capable of selectively binding to stem cell surface markers. The advantage of this type of formulation is to enhance proximity between the microbubble and cell surface so that the ultrasound-stimulated effects are amplified and enhanced.

The pharmaceutical composition according to the present invention additionally contains target cells for the pharmaceutically active nucleic acid, wherein the target cells are primary cells, expanded cells or a stromal cell fraction, especially fibroblasts, bone progenitor cells or adult mesenchymal stem cells.

"Primary cells" are defined as cells obtained using primary cultures, which are cultures prepared directly from the tissues of an organism without cell proliferation in vitro (expansion). "Expanded cells" are cells in a secondary culture, which are derived from primary cell cultures and undergo in vitro expansion by proliferation (for both definitions see: Alberts et al., in: S. Gibbs, eds, "Molecular Biology of the Cell" (2002), 472; Garland Science, New York). "Stromal cells" are defined as fibroblastic cells from bone marrow with self-renewing, multipotent character, capable of differentiation to mesenchymal tissues such as bone, cartilage or fat cells and are therefore also referred to as mesenchymal stem cells (definition in: Alberts et al., in: S. Gibbs, eds, "Molecular Biology of the Cell" (2002), 1300; Garland Science, New York).

Although the composition according to the present invention may be used to transform cells already present in a patient, target cells are administered together with the matrix or the matrix precursor. This specifically advantageous if the matrix forms a scaffold for the new tissue to be generated by the compositions according to the present invention. In this case, cells do not need to actively invade the matrix but can be provided at the selected density and with the desired cell specificity. After administration of a cell containing matrix (or after formation of the cell containing matrix), a sufficient time can be waited before applying ultrasound for transfection of the cells with the nucleic acid. This provides the combined advantages of holding the target cell population where it is required (i.e. in the cavity of a non-union fracture) and further provides advantage by allowing the target cell population to become accustomed to it new environment. Preferably, the target cells used in the pharmaceutical compositions are autologous cells of the individual to whom the compositions according to the present invention are administered.

In preferred pharmaceutical compositions according to the present invention, the target cells comprise primary cells or primary stromal cells (e.g. fibroblasts, stem cells), where the cells are harvested and re-implanted to the patient preferably in a one-step procedure.

The nature of the nucleic acid to be applied to the target cells can be quite diverse. DNA, RNA, RNA-DNA hybrid molecules, modified DNA or RNA (e.g. phosphorothioate forms of DNA or RNA or nucleic acids with a 3-deazapurine ring system), PNA, and any combination of these nucleic acids can be applied. However, the specifically preferred nucleic acid according to the present invention is - also due to its stability - DNA, preferably a plasmid, a minicircle, a linearised molecule or mixtures thereof. These molecules applied contain - besides the nucleic acid to be expressed in the cells - also the necessary sequences allowing a proper action of the nucleic acid molecule inside the cell, such as regulators (promoters, terminators), replication origins, markers, integration sequences, etc.. Also DNA modifications leading to further stabilisation, such as phosphorothioate and 3-deazapurine forms of DNA are preferred.

Specifically preferred nucleic acids in the pharmaceutical composition according to the present invention include: plasmid DNA (pDNA) or minicircle pDNA: encoding
A) plasmid DNA (pDNA), minicircle pDNA or *in vitro* transcribed messenger RNA (mRNA) encoding reading frames of therapeutic genes required for the induction of cellular differentiation, cell migration, the expression anti-microbial peptides and anti-inflammatory chemokines mediating tissue regeneration;
B) plasmid DNA (pDNA), minicircle pDNA
shRNA, microRNAs required for the down-regulation of relevant target genes either up-regulated in the diseases state that require down-regulation for tissue regeneration or targeting genes that impair the cellular differentiation response required for tissue regeneration.

Moreover, other preferred nucleic acids according to the present invention are therapeutic RNA molecules, such as synthetic siRNA, microRNA, locked nucleic acids (LNAs; e.g. Kuppinen et al., 2005; and the literature cited therein),) employed for gene knockdown (down-regulation) of aberrantly expressed or up-regulated genes under pathologic conditions that impair tissue regeneration/cellular differentiation.

Also transposon-based systems (e.g. Sleeping Beauty) and/or combinations of the aforementioned DNAs/RNAs with the appropriate enhancer/promoter sequences to facilitate expression of the relevant transgene are appropriate. Constructs are em-employed which facilitate transient short or long-term expression of the transgene. In addition, the transgene may comprise any genetic component capable of stem cell morphogenesis and/or modulation such that tissue regeneration is enabled.

According to a preferred embodiment, the present pharmaceutical composition contains a pharmaceutically active nucleic acid, which encodes a therapeutic gene.

A "therapeutic gene" is defined herein as a eukaryotic transcriptional unit based on DNA or a synthetic eukaryotic translational unit based on RNA that upon intracellular delivery drives the expression of the gene of interest (i.e. a cytokine, intracellular signal transducer or transcription factor), which in turn induces the desired differentiation stimulus. Furthermore, also a synthetic double stranded short interfering RNA (siRNA) of 21-25mer length or a transcriptional unit driving the RNA-Polymerase II or RNA-Polymerase III based expression of short hairpin RNA (shRNA), which in turn mediates the RNA-interference based down regulation of any desired target gene (both approaches described in Grimm, Adv. Drug Deliv. Rev. 61 (2009), 672-703) in order to induce the desired differentiation stimulus, can be a therapeutic gene according to the present invention. In case of a DNA-based transcriptional unit, the therapeutic gene encompasses any double stranded linear or circular deoxyribonucleic acid sequence in 5' to 3' direction, which possesses the following features (in 5' to 3' order, described in: Tolmachov, in: Walther et al., eds., "Methods in Molecular Biology, Gene Therapy of Cancer" (2009), p119-120, Springer):
- an enhancer element, enhancing the promoter (see below) based transcription;
- a promoter element triggering RNA-Polymerase II dependent initiation and transcription of eukaryotic messenger RNA;
- an open reading frame (ORF) for a cytokine, intracellular signal transducer or transcription factor with a Kozak sequence for efficient translation initiation an eukaryotic start codon, the triplet based coding sequence of the amino acid sequence of the gene of interest (i.e a cytokine, intracellular messenger or transcription factor) including an eukaryotic stop codon; and
- an efficient transcriptional terminator sequence and polyadenylation signal for mRNA processing.

Two such transcriptional units can be furthermore combined in a modular way within one DNA molecule to drive the bidirectional (shared promoter element) or separate (two independent transcriptional units) expression of 2 open reading frames simultaneously.

In case of a synthetic RNA based translational unit, the therapeutic gene encompasses any single stranded linear ribonucleic acid in 5' to 3' direction, which possesses the following features (as described in: Yamamoto et al., Eur. J. Pharm. and Biopharm. 71 (2009), 484-489; Tavernier et al., J. Contr. Rel. 150 (2011), 238-247; Alberts et al., in: S. Gibbs, eds. "Molecular Biology of the Cell" (2002), 689-709; Garland Science, New York):
- a 5' cap structure containing either a 7-methylguanosine nucleotide or equivalent derivative;
- a 5' untranslated region (5' UTR) of sufficient length for efficient messenger RNA mimicking including a Kozak sequence for initiation of translation;
- an open reading frame (ORF) for a cytokine, intracellular signal transducer or transcription factor with a Kozak sequence for efficient initiation of translation, an eukaryotic start codon, the triplet based coding sequence of the amino acid sequence of the cytokine, intracellular messenger or transcription factor including an eukaryotic stop codon; and
- a 3' untranslated region (3' UTR) of sufficient length for efficient messenger RNA mimicking including elements mediating enhanced RNA half-life and a poly-adenosine sequence of at least 100 terminal adenosines at the 3' end.

Preferrred therapeutic genes according to the present invention are osteogenic and/or angiogenic factors, e.g. BMPs, VEGF and PDGF and/or released cytokines that exert autocrine and paracrine differentiation stimuli.

An "osteogenic factor" is defined as a protein being capable of triggering the osteogenic differentiation of target cells trough autocrine and paracrine ligand (encoded cytokine) - receptor interaction mediated signal transduction. Examples for such osteogenic factors are translated protein products of an open reading frame for a cytokine from the TGFß- growth factor family such as TGFß3, Bone morphogenetic proteins (BMPs) 2,4,6 and 7; a WNT (wingless/Int-1)-family cytokine such as WNT5a and WNT7a; Furthermore, an osteogenic factor can be a transcription factor involved in osteogenic differentiation such as osterix (OSX) or the runt related transcription factor 2 (Runx2)/core-binding factor subunit alpha-1 (CBF-alpha-1) triggering the expression of osteogenic target genes and enabling osteogenic differentiation in target cells (BMP Buch, BMP/WNT reviews, etc. further examples are disclosed in Deng et al., Front. Biosci. 13 (2008), 2001-2021; Kofron et al., Adv. Drug Del. Rev. 58 (2006), 555-576).

An "angiogenic factor" is defined as a protein triggering angiogenesis and vasculogenesis. Examples for such angiogenic factors are translated protein products of an open reading frame for a cytokine from the vascular endothelial growth factor family such as VEGF-A and (placenta growth factor) PIGF; or the fibroblast growth factor family, i.e. basic fibroblast growth factor (bFGF/FGF2). Furthermore, an angiogenic factor can be any translated protein product of an open reading frame for a cytokine mediating stability (stabilizing or preventing regression) of newly formed vessels such as platelet derived growth factor B (PDGFB) or angiopoietin 1 and 2 (Ang1, Ang2). Further examples are disclosed in: Sedighiani et al., Surgeon 9 (2011), 326-335; Lei et al., Basic Res. Cardiol. 99 (2004), 121-132).

According to another aspect, the present invention also relates to a method for treating a patient comprising administering a pharmaceutical composition according to the present invention, and applying ultrasound to introduce the pharmaceutically active nucleic acid into cells in the patient (to trigger constitutive or regulated transgene expression) whereafter the active nucleic acid is expressed or is caused to be expressed in said cells. If the nucleic acid applied contains a constitutive promoter, the gene is immediately active after transduction by sonoporation. If an inducible promoter is provided with the nucleic acid, the gene transferred to the cells may be separately switched on and off by the inducible promoter system.

The application of ultrasound to a specific location in the patient is a technique which is known in principle to the skilled person (see e.g. Li et al., Newman et al., Mehier-Humbert et al., US 7,341,569 A, US 2001/0031740 A1, etc.). Preferably, ultrasound is used as an extracorporeal stimulus for gene transfection, since it can be delivered non-invasively at any time post-surgical insertion of the matrix. In terms of the ultrasound that may be employed in such a system, e.g. a generator may emit a single columnar ultrasound beam or alternatively emit a number of columnar beams to facilitate enhanced energy deposition at a single point at depth in tissues/matrices. An alternative to the latter approach is to employ a focused transducer and this would achieve a similar result. Preferably, the ultrasound is therefore emitted from a single transducer as a columnar beam, from more than one transducer providing an intersecting beam configuration or from a focused transducer. The ultrasound may also be applied via a catheter (see e.g. US 7,341,569 A and further US-patents cited therein).

Preferably, the ultrasound is operated at a frequency from 15 kHz to 10 MHz, more preferred from 15 kHz to 5 MHz, especially from 500 kHz to 3 MHz, for transforming the target cells with the pharmaceutically active nucleic acid. A preferred example of a sonoporation platform is the Sonidel platform (e.g. the SONIDEL SP100; 1 MHz output frequency; intensity adjustable between 0 and 5 W/cm² and incorporating a 100 Hz pulse delivery frequency with variable duty cycle).

The present invention is broadly applicable in all fields of non-viral in situ gene therapies. A preferred embodiment is bone tissue regeneration, however, also e.g. tumour patients can be efficiently treated according to the present invention or patients which have undergone major surgery of the lung, liver or heart.

According to a preferred embodiment, the pharmaceutical composition according to the present invention is applied for bone tissue regeneration. Preferably, exogenous genes are applied according to the present invention which control morphogenesis of the target cells. The matrix according to the present invention (or the matrix to be formed in situ according to the present invention) then acts as a biocompatible bone scaffold allowing a true functional replacement.

According to a preferred embodiment of the present invention in bone regeneration, the pharmaceutically active nucleic acid encodes a bone morphogenic protein (BMP; a group of growth factors and cytokines inducing the formation of bone and cartilage; currently about 20 different BMPs are known), especially BMP-1, BMP-2, BMP-3, BMP-4, BMP-5, BMP-6, BMP-7 and BMP-8; and/or an angiogenic factor, especially acidic or basic FGF, a mixture of basic FGF and heparin sulfate, TGF-beta, PDGF-alpha, angiogenin, angiotropin, CD-RAP/MIA; and combinations thereof. If BMP-encoding genes are employed, this has the net effect of inducing the formation of bone thereby replacing the skeletal deficit. If angiogen(et)ic factor-encoding genes are employed, these have the net effect of inducing the formation of a vascular network throughout the matrix, especially to enhance perfusion and resulting tissue growth.

In this specific field, bone-compatible matrices or matrix precursors are preferred which are suitable for being placed in contact with bone tissue, such as the materials disclosed in US 5,270,300 A (collagen, fibrin, gelatin, agarose, etc.).

In this embodiment, it is advantageous to use genes to stimulate bone progenitor cells and/or bone progenitor cells as target cells. Bone progenitor cells are those cells that have the capacity to ultimately form, or contribute to the formation of, new bone tissue. This includes various cells in different stages of differentiation, such as, for example, stem cells, macrophages, fibroblasts, vascular cells, osteoblasts, chondroblasts, osteoclasts, and the like. Bone progenitor cells also include cells that have been isolated and manipulated in vitro, e.g., subjected to stimulation with agents such as cytokines or growth factors or even genetically engineered cells. The particular type or types of bone progenitor cells that are stimulated using the methods and compositions of the invention are not important, so long as the cells are stimulated in such a way that they are activated and, in the context of in vivo embodiments, ultimately give rise to new bone tissue.

In using the present invention in bone regeneration, suitable embodiments are also disclosed in US 5,270,300 A and US 5,763,416 A. Typical indications to be treated according to the present invention are bone defects, osteoporosis, osteoarthritis or osteogenesis imperfecta. Such bone defects include bone frac-fractures, joint fractures, non-unions and delayed unions, percutaneous arthrodesis, pseudo-arthrosis and bone defects resulting from congenital defects, trauma, tumour infection, degenerative disease and other causes of loss of skeletal tissue. The bone repairing matrix compositions according to the present invention are also useful for prosthesis implantation and enhancement of prosthesis stability, enhancement of osseointegration of implant materials used for internal fixation procedures, stabilization of dental implant materials, healing acceleration of ligament insertion, and spine or other joint fusion procedures; and enhancement of distraction osteogenesis.

The invention is further illustrated by the following examples and the drawings, yet without being restricted thereto.
Fig.1 shows luciferase expression in mouse hind-leg muscle using three different DNA constructs; and
Fig.2 shows luciferase expression in matrices treated with ultrasound in the absence of microbubbles (+U/S -MB), in the presence of microbubbles without ultrasound treatment (-U/S +MB) and in both the presence of microbubbles and ultrasound irradiation (+U/S +MB) for the fibrin-based matrices, containing collagen (+collagen) or not (-collagen). Error bars represent + SEM where n = 5;

### EXAMPLES

### Example (comparative example)

### Non-invasive gene transfection and control of longevity of transgene expression by manipulation of matrix formulation components:

In order to demonstrate that ultrasound may be employed to extracorporeally trigger gene transfection in a tissue (natural matrix) harbouring the components of the invention (i.e. target cells, microbubbles and nucleic acid), it was decided to inject these components into the hind-leg muscle of isofluorane-anaesthetised mice. The target cells embedded in their natural matrix in situ (the muscle), obviously represent a environment comparable to the devised constructs. Injections volumes were 40µl and consisted of a 1:1 mix of MB101 (Sonidel Ltd.) microbubbles and the relevant transgene-encoding DNA at a concentration of 1µg/µl. Microbubbles were therefore employed at a final concentration of 4x10⁸ microbubbles/ml. Immediately following injection the transducer of a SP100 sonoporator (Sonidel Ltd.) was placed in contact with the skin (via application of ultrasound gel) overlying the injection site and that site was treated with ultrasound at a frequency of 1MHz, an intensity of 1.9 W/cm² (SATP) and using a duty cycle of 25% for 3 min. Following transfection, animals were permitted to recover from anaesthesia and transgene expression (luciferase) was monitored by in vivo imaging using a Xenogen IVIS_ Lumina imaging system supported by Living Image_ software version2.60.

### Results

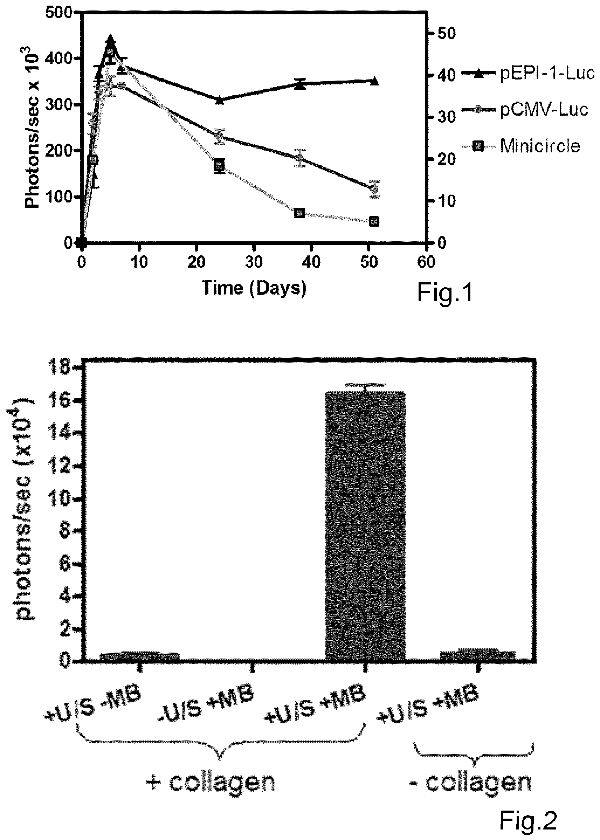

In these experiments the objective was to demonstrate the advantages in terms of using ultrasound as a stimulus to facilitate gene transfection non-invasively using the components of the matrix formulation and using a non-surgically exposed target. In these experiments also, it was decided to examine the possibility of using different types of DNA in the matrix formulation in order to facilitate either long-term, medium-term and short-term expression of a transgene in the target cells. To these latter ends the plasmid pEPI-1-Luc was employed to facilitate long term expression, exploiting the existence of S/MAR sequences in this construct. To achieve medium term transgene expression the conventional reporter plasmid, pCMV-Luc was employed. In order to examine the possibility of using minicircle DNA, since this does not contain DNA derived from bacterial sources and is therefore more applicable for use as a therapeutic, the PlasmidFactory Minicircle DNA (MC07.CMV-Luc) was employed. As mentioned above, expression of luciferase was monitored over a 51-day period and it was found that prolonged expression of luciferase was achieved using the plasmid pEPI-1-Luc. When the reporter plasmid, pCMV-Luc was employed, expression peaked between 2 and 8 days and then began to reduce significantly towards day 51. When the minicircle DNA was employed there was expression of the luciferase in the target cells (see Fig.1, right Y-axis) and expression was found to peak at day 5. The level of expression then reduced rapidly to a minimum between days 38 and 51. These results demonstrate the advantage of the overall mixture of matrix formulation components together with ultrasound in achieving non-invasive expression of an exogenous transgene. The results further demonstrate advantage of the invention in terms of the adaptability of the system in choosing variations in those components to facilitate either short-term and/or long term expression of a transgene and demonstrate flexibility and enhanced control of this parameter for therapeutic purposes. In addition, the results also demonstrate advantage in terms of being able to employ minicircle DNA, a construct that has been shown to be more suitable for gene therapy as a result of its lack of bacterial DNA (Zhang et al.).

### Example 2.

### Ultrasound-mediated stimulation of gene transfer into target cells in a fibrin-collagen matrix containing microbubbles.

In order to demonstrate that ultrasound could be employed to facilitate gene transfer in a gel-based matrix, 2 fibrin-based matrices were employed. One of those comprised fibrin alone and the other comprised fibrin plus collagen type I. C2C12 cells were suspended in OptiMEM. Cells were mixed with microbubbles (SDM302 - biotinylated microbubbles). DNA was added to this mixture. For fibrin-collagen matrices, neutralised collagen solution was subsequently added to the above mixture. Finally, fibrinogen solution, supplemented with 5x OptiMEM medium (10% v/v) was added to the above suspension. A given volume of the above mixtures (40 µl) was added to an equal volume of thrombin solution also supplemented with 5X OptiMEM (50% v/v) and the mixture was dispensed rapidly into a well of a 96 well plate in order to solidify. Gels comprised 22 mg/ml fibrin and where collagen was used, this was at a final concentration of 0.3 mg/ml in gels. Each gel matrix contained 6 x 10⁴ cells per well. The final concentrations of microbubbles and DNA in each gel were 4.8 x 10⁷ microbubbles per ml and 20 µg of DNA per ml. In the absence of collagen or microbubbles, volumes equivalent to the latter additions were replaced by PBS. Each well was then treated with ultrasound for 30 s at an ultrasound frequency of 1MHz, a power density of 4 W/cm², using a duty cycle of 25% (pulse frequency = 100 Hz). Following treatment, 150 µL of OptiMEM medium was added per well and plates were incubated for 1h at 37°C in a humidified 5% CO₂ atmosphere. OptiMEM was then replaced with DMEM (high glucose) medium containing 5% (v/v) foetal bovine serum and plates were incubated 24 h prior to analysis. Gene expression was quantified following exposure of gels to luciferin and subsequent imaging using a Xenogen IVIS^{®} Lumina imaging system supported by Living Image^{®} software version 2.60.

### Results

The results obtained for gene expression during these studies are shown in Fig.2. In the absence of ultrasound, no luciferase activity was present and this indicated that no gene transfer had occurred. Similarly, in the absence of microbubbles only a trace of luciferase was detected, again indicating that little or no gene transfer had occurred. Interestingly however in the absence of collagen and in the presence of microbubbles and ultrasound little or no luciferase activity was detected and this again suggested that little or no gene transfer was occurring in the gels. This was a surprising result because to those familiar with the art and from our previous studies (Nomikou *et al.;* Li *et al*.) there was every reason to expect gene transfer to have occurred in this system. It had been demonstrated by Nomikou *et al*. that use of the specific microbubble SDM302 would ensure direct contact between microbubbles, DNA and target cells within the matrix, thereby ensuring sonoporation. Indeed this strategy was specifically employed to preclude any challenges presented by the lack of mobility of components such as microbubbles, DNA and target cells within the gel matrix. However, during these studies we discovered that inclusion of collagen in the fibrin gels enabled gene transfer to occur and these data are shown in Fig.2. Once again, this was a surprising discovery and the controls indicated that gene transfer was not due to some mixture of components in the gel because when either ultrasound or microbubbles were omitted from those gels, no gene transfer occurred. Therefore, this unique discovery clearly demonstrates that formulations comprising a mixture of fibrin and collagen together with the appropriate microbubble type can be mixed in the above-described sequence to enable ultrasound-mediated, microbubble-enhanced gene transfer in 3-D gel matrices and further suggests that this strategy can be employed as a therapeutic formulation/methodology for disease treatment as suggested in the background.

### Example 3

### Ultrasound-mediated transfection of target stem cells in the matrix formulation with bone formation in vivo

Since the above Example 2 had demonstrated that ultrasound could be employed to facilitate gene transfer in fibrin/collagen gels and in order to demonstrate that matrix assisted sonoporation of target cells embedded within the matrix can trigger differentiation and subsequent tissue formation within the matrix depending on the applied transgene, it was decided to introduce the above-described mixtures into animals at specific target sites and determine whether or not gene transfer could be stimulated within those gels an using extracorporeal ultrasound stimulus. Therefore the bone morphogenetic protein encoding genes BMP2 and BMP7 were selected as therapeutic genes to be co-delivered through a co-expression plasmid within this setup since they are known to potently induce bone formation if delivered by gene transfer approaches (Kawai et al., Osawa et al.). Autologous fibroblasts from Sprague-Dawley rats were used as target cells. These cells were used at a concentration of 1-10⁶/ml within fibrin/collagen matrices as described above for Example 2. The employed final plasmid concentration was 80µg/ml. SDM302 microbubbles were provided at a concentration of 4x10⁸ particles/ml. The clots containing the cells, microbubbles and plasmid DNA were prepared analogous to Experiment 2. The clots were incubated for 30min at 37°C, 5% CO₂ prior to implantation. Clot size per animal was 200µl fibrin clot with a diameter of approx. 0,8cm and a height of 4mm.

Male Sprague Dawley rats were anaesthetised using isofluorane and the constructs were implanted either into the hind-leg muscle or subcutaneously into the back. Clots containing only DNA and cells served as negative controls. Sonoporation using the SP100 1MHz sonoporator was carried out transdermally after suturing of the wounds. The sonoporation parameters used were 1.9W/cm², 25% duty cycle and 3min of exposure using an ultrasound contact gel and an ultrasound adsorber pad. The implants were imaged by in vivo µCT at 4 weeks, 6 weeks and 8 weeks after sonoporation and harvested at 8 weeks for analysis. Mineralization was assessed by von Kossa staining additionally to standard HE tissue histology and alkaline phosphatase activity (osteoblast marker) was assessed by enzymatic assays.

### Results

Induction of bone formation at heterotopic sites through matrix assisted sonoporation of target cells with BMP2/BMP7 co-expression plasmids was the aim of this experiment in order to demonstrate the overall feasibility of the approach for tissue engineering and especially for bone regeneration approaches. The formation of viable mineralized bone tissue is the main parameter for the success of the experiment.

Significant bone formation compared to the negative controls was observed in all animals at 8 weeks after surgery and sonoporation. Bone formation/osteoblast differentiation was confirmed by in vivo µCT measurements, Histology, von Kossa staining and ALP-assays. Overall it has been demonstrated that the approach of matrix assisted sonoporative gene transfer of osteoinductive genes to embedded autologous cells is capable of triggering the generation of the desired tissue type in vivo. Therefore, it is demonstrated that the intended invention is applicable for the ultrasound-triggered formation of bone in vivo in order to replace or regenerate injured tissue. This example also demonstrates advantage in terms of being capable of positioning and retaining the target cells and transfection machinery at the target site. For example, if such an approach was taken in the absence of a matrix, then the transfection machinery and indeed the target cells would tend to diffuse away from the target site and less positional control would be exerted on the therapeutic transfection events and consequences thereof. Although ultrasound-mediated gene transfer is possible in fibrin-based matrices, our discovery demonstrates that the addition of collagen very significantly enhances gene transfer in those fibrin matrices.

### References:

Alberts et al., in: Gibbs, eds, "Molecular Biology of the Cell" (2002), 472; Garland Science, New York.
Alberts et al., in: Gibbs, eds, "Molecular Biology of the Cell" (2002), 1300; Garland Science, New York.
Alberts et al., in: Gibbs, eds. "Molecular Biology of the Cell" (2002), 689-709; Garland Science, New York.
Chen et al., Med Oncol. 7 (2009), DOI 10.1007/s12032-008-9161-0.
Christiansen et al., Ultrasound Med. Biol. 29 (2003), 1759 1767. Deng et al., Front. Biosci. 13 (2008), 2001-2021.
Farook et al., J. R. Soc. Interface 6 (2009), 271-277.
Ferguson et al., p.226; in: von Byern et al., eds.; "Biological Adhesive Systems: From Nature to Technical and Medical Application" (2010), Springer.
Grimm, Adv. Drug Deliv. Rev. 61 (2009), 672-703 Kawai et al. BMC Musculoskeletal Disord. 7 (2006) 1471-2474
Kofron et al., Adv. Drug Del. Rev. 58 (2006), 555-576.
Kuppinen et al., Drug Discov. Today 2 (2005), 287-290.
Lei et al., Basic Res. Cardiol. 99 (2004), 121-132.
Li et al., Cancer Letters 273 (2009), 62-69.
Macaya et al., Adv. Funct. Mat. 21 (2011), 4788-4797.
Mehier-Humbert et al., Adv Drug Deliv. 57 (2005), 733-753.
Newman et al., Gene Therapy 14 (2007), 465-475.
Nomikou et al., Acta Biomaterialia, 8 (2012) 1273-1280.
Nürnberger et al., p.240; in: von Byern et al., eds.; "Biological Adhesive Systems: From Nature to Technical and Medical Application" (2010), Springer.
Osawa et al. J Gene Med. 11 (2009), 633-641.
Palmer et al., J. Orthop. Res. 27 (2011), 964-971.
Rapoport et al., Bubble Sci. Eng. Technol. 1 (2009), 31-39. Saito et al., J Orthop Res. 25 (2007), 1308-1316.
Schek et al., Eur. Cells Mat. 21 (2011), 373-383.
Sedighiani et al., Surgeon 9 (2011), 326-335.
Stride et al., Med. Biol. Eng. Comput. 47 (2009), 883-892.
Tavernier et al., J. Contr. Rel. 150 (2011), 238-247.
Tolmachov, in: Walther et al., eds., "Methods in Molecular
Biology, Gene Therapy of Cancer" (2009), p119-120, Springer. Torre et al., Intl. J. Pharmaceutics 242 (2009), 389-391. Yamamoto et al., Eur. J. Pharm. Biopharm. 71 (2009), 484-489. Zhang et al., Human Gene Ther. 19 (2008), 820-826.

## Claims

1. : Pharmaceutical composition comprising
- a soluble matrix precursor, wherein the soluble matrix precursor is an alginate, collagen, hyaluronic acid, fibrinogen, gelatin, chondroitin sulfate or combinations thereof,
- an ultrasound transfection agent,
- a pharmaceutically active nucleic acid, and
- target cells for the pharmaceutically active nucleic acid, wherein the target cells are primary cells, expanded cells or a stromal cell fraction, especially fibroblasts, bone progenitor cells or adult mesenchymal stem cells.

2. : Pharmaceutical composition according to claim 1 **characterised in that** the soluble matrix precursor is a liquid hydrogel base.

3. : Pharmaceutical composition according to claim 1 or 2, **characterised in that** it further comprises a matrix activator.

4. : Pharmaceutical composition according to any one of claims 1 to 3, **characterised in that** it is provided in a set form comprising
a) a soluble matrix precursor component,
b) a matrix activator component, and
c) target cells for the pharmaceutically active nucleic acid, preferably primary cells, expanded cells or a stromal cell fraction, especially fibroblasts, bone progenitor cells or adult mesenchymal stem cells,
wherein the ultrasound transfection agent and the pharmaceutically active nucleic acid are contained either in a) or in b) or in both, preferably in a).

5. : Pharmaceutical composition comprising
- a matrix, wherein the matrix is solidified alginate, solidified collagen, solidified hyaluronic acid, fibrin, solidified gelatin, solidified chondroitin sulfate or mixtures thereof,
- an ultrasound transfection agent, and
- a pharmaceutically active nucleic acid, and
- target cells for the pharmaceutically active nucleic acid, wherein the target cells are primary cells, expanded cells or a stromal cell fraction, especially fibroblasts, bone progenitor cells or adult mesenchymal stem cells.

6. : Pharmaceutical composition according to any one of claims 1 to 5, **characterised in that** the ultrasound transfection agent is selected from microbubbles, nanobubbles, nanodroplets or mixtures thereof.

7. : Pharmaceutical composition according to any one of claims 1 to 6, **characterised in that** the target cells are fibroblasts.

8. : Pharmaceutical composition according to any one of claims 1 to 7, **characterised in that** the pharmaceutically active nucleic acid is a DNA molecule, preferably a plasmid, a minicircle, a linearised molecule or mixtures thereof.

9. : Pharmaceutical composition according to any one of claims 1 to 8, **characterised in that** the soluble matrix precursor is a mixture of collagen and fibrinogen.

10. : Pharmaceutical composition according to any one of claims 1 to 9 for use in a method for treating a patient comprising administering the composition to the patient and applying ultrasound to introduce the pharmaceutically active nucleic acid into cells in the patient whereafter the active nucleic acid is expressed or is caused to be expressed in said cells.

11. : Pharmaceutical composition according to claim 10, **characterised in that** the ultrasound is emitted from a single transducer as a columnar beam, from more than one transducer providing an intersecting beam configuration or from a focused transducer.

12. : Pharmaceutical composition according to claim 10 or 11, **characterised in that** the ultrasound is operated at a frequency from 15 kHz to 10 MHz, especially from 15 kHz to 5 MHz.

13. : Pharmaceutical composition according to any one of claims 10 to 12, **characterised in that** the composition is administered to the patient whereafter a physical step is performed for converting the soluble matrix precursor to a matrix.

## Patentansprüche

1. Pharmazeutische Zusammensetzung, umfassend
- eine lösliche Matrixvorstufe, bei der es sich um Alginat, Kollagen, Hyaluronsäure, Fibrinogen, Gelatine, Chondroitinsulfat oder Kombinationen aus diesen handelt,
- ein Ultraschalltransfektionsmittel,
- eine pharmazeutisch aktive Nukleinsäure, und
- Zielzellen für die pharmazeutisch aktive Nukleinsäure, bei denen es sich um primäre Zellen, expandierte Zellen oder eine stromale Zellfraktion, insbesondere Fibroblasten, Knochenvorläuferzellen oder adulte mesenchymale Stammzellen handelt.

2. Pharmazeutische Zusammensetzung nach Anspruch 1, **dadurch gekennzeichnet, dass** es sich bei der löslichen Matrixvorstufe um eine flüssige Hydrogelbasis handelt.

3. Pharmazeutische Zusammensetzung nach Anspruch 1 oder 2, **dadurch gekennzeichnet, dass** diese des Weiteren einen Matrixaktivator umfasst.

4. Pharmazeutische Zusammensetzung nach einem der Ansprüche 1 bis 3, **dadurch gekennzeichnet, dass** diese in Form eines Sets bereitgestellt wird, das umfasst:
a) eine lösliche Matrixvorstufenkomponente,
b) eine Matrixaktivatorkomponente, und
c) Zielzellen für die pharmazeutisch aktive Nukleinsäure, bevorzugt primäre Zellen, expandierte Zellen oder eine stromale Zellfraktion, insbesondere Fibroblasten, Knochenvorläuferzellen oder adulte mesenchymale Stammzellen,
wobei das Ultraschalltransfektionsmittel und die pharmazeutisch aktive Nukleinsäure entweder in a) oder in b) oder in beiden, vorzugsweise in a), enthalten sind.

5. Pharmazeutische Zusammensetzung, umfassend
- eine Matrix, bei der es sich um verfestigtes Alginat, verfestigtes Kollagen, verfestigte Hyaluronsäure, Fibrin, verfestigte Gelatine, verfestigtes Chondroitinsulfat oder Gemischen aus diesen handelt
- ein Ultraschalltransfektionsmittel,
- eine pharmazeutisch aktive Nukleinsäure, und
- Zielzellen für die pharmazeutisch aktive Nukleinsäure, bei denen es sich um primäre Zellen, expandierte Zellen oder eine stromale Zellfraktion, insbesondere Fibroblasten, Knochenvorläuferzellen oder adulte mesenchymale Stammzellen handelt.

6. Pharmazeutische Zusammensetzung nach einem der Ansprüche 1 bis 5, **dadurch gekennzeichnet, dass** das Ultraschalltransfektionsmittel aus Mikrobläschen, Nanobläschen, Nanotröpfchen oder Gemischen aus diesen ausgewählt ist.

7. Pharmazeutische Zusammensetzung nach einem der Ansprüche 1 bis 6, **dadurch gekennzeichnet, dass** es sich bei den Zielzellen um Fibroblasten handelt.

8. Pharmazeutische Zusammensetzung nach einem der Ansprüche 1 bis 7, **dadurch gekennzeichnet, dass** es sich bei der pharmazeutisch aktiven Nukleinsäure um ein DNA-Molekül, bevorzugt ein Plasmid, einen Minicircle, ein linearisiertes Molekül oder Gemische aus diesen handelt.

9. Pharmazeutische Zusammensetzung nach einem der Ansprüche 1 bis 8, **dadurch gekennzeichnet, dass** es sich bei der löslichen Matrixvorstufe um ein Gemisch aus Kollagen und Fibrinogen handelt.

10. Pharmazeutische Zusammensetzung nach einem der Ansprüche 1 bis 9 zur Verwendung in einem Verfahren zur Behandlung eines Patienten, umfassend das Verabreichen der Zusammensetzung an den Patienten sowie das Applizieren von Ultraschall zum Einbringen der pharmazeutisch aktiven Nukleinsäure in Zellen des Patienten, woraufhin die aktive Nukleinsäure in diesen Zellen exprimiert wird bzw. deren Expression in diesen Zellen bewirkt wird.

11. Pharmazeutische Zusammensetzung nach Anspruch 10, **dadurch gekennzeichnet, dass** der Ultraschall als säulenförmiger Strahl von einem einzigen Ultraschallkopf, in einer Konfiguration mehrerer sich kreuzender Strahlen von mehr als einem Ultraschallkopf oder von einem fokussierten Ultraschallkopf emittiert wird.

12. Pharmazeutische Zusammensetzung nach Anspruch 10 oder 11, **dadurch gekennzeichnet, dass** der Ultraschall bei einer Frequenz von 15 kHz bis 10 MHz, insbesondere von 15 kHz bis 5 MHz, emittiert wird.

13. Pharmazeutische Zusammensetzung nach einem der Ansprüche 10 bis 12, **dadurch gekennzeichnet, dass** die Zusammensetzung an den Patienten verabreicht wird, woraufhin ein physikalischer Schritt zur Umwandlung der löslichen Matrixvorstufe in eine Matrix durchgeführt wird.

## Revendications

1. Composition pharmaceutique comprenant
- un précurseur de matrice soluble, le précurseur de matrice soluble étant un alginate, un collagène, de l'acide hyaluronique, un fibrinogène, une gélatine, du sulfate de chondroïtine ou des combinaisons de ceux-ci,
- un agent de transfection par ultrasons,
- un acide nucléique pharmaceutiquement actif, et
- des cellules cibles pour l'acide nucléique pharmaceutiquement actif, les cellules cibles étant des cellules primaires, des cellules expansées ou une fraction de cellules stromales, en particulier des fibroblastes, des cellules progénitrices osseuses ou des cellules souches mésenchymateuses adultes.

2. Composition pharmaceutique selon la revendication 1, **caractérisée en ce que** le précurseur de matrice soluble est une base d'hydrogel liquide.

3. Composition pharmaceutique selon la revendication 1 ou 2, **caractérisée en ce qu'**elle comprend en outre un activateur de matrice.

4. Composition pharmaceutique selon l'une quelconque des revendications 1 à 3, **caractérisée en ce qu'**elle est fournie sous une forme fixe comprenant
a) un composant précurseur de matrice soluble,
b) un composant activateur de matrice, et
c) des cellules cibles pour l'acide nucléique pharmaceutiquement actif, de préférence des cellules primaires, des cellules expansées ou une fraction de cellules stromales, en particulier des fibroblastes, des cellules progénitrices osseuses ou des cellules souches mésenchymateuses adultes,
dans laquelle l'agent de transfection par ultrasons et l'acide nucléique pharmaceutiquement actif sont contenus dans a) ou dans b) ou dans les deux, de préférence dans a).

5. Composition pharmaceutique comprenant
- une matrice, la matrice étant un alginate solidifié, un collagène solidifié, de l'acide hyaluronique solidifié, de la fibrine, une gélatine solidifiée, du sulfate de chondroïtine solidifié ou des mélanges de ceux-ci,
- un agent de transfection par ultrasons,
- un acide nucléique pharmaceutiquement acceptable, et
- des cellules cibles pour l'acide nucléique pharmaceutiquement actif, les cellules cibles étant des cellules primaires, des cellules expansées ou une fraction de cellules stromales, en particulier des fibroblastes, des cellules progénitrices osseuses ou des cellules souches mésenchymateuses adultes.

6. Composition pharmaceutique selon l'une quelconque des revendications 1 à 5, **caractérisée en ce que** l'agent de transfection par ultrasons est choisi parmi les microbulles, les nanobulles, les nanogouttelettes, ou des mélanges de ceux-ci.

7. Composition pharmaceutique selon l'une quelconque des revendications 1 à 6, **caractérisée en ce que** les cellules cibles sont des fibroblastes.

8. Composition pharmaceutique selon l'une quelconque des revendications 1 à 7, **caractérisée en ce que** l'acide nucléique pharmaceutiquement actif est une molécule d'ADN, de préférence un plasmide, un minicercle, une molécule linéarisée ou des mélanges de ceux-ci.

9. Composition pharmaceutique selon l'une quelconque des revendications 1 à 8, **caractérisée en ce que** le précurseur de matrice soluble est un mélange de collagène et de fibrinogène.

10. Composition pharmaceutique selon l'une quelconque des revendications 1 à 9, pour une utilisation dans une méthode de traitement d'un patient comprenant l'administration de la composition au patient et l'application d'ultrasons pour introduire l'acide nucléique pharmaceutiquement actif dans les cellules du patient, après quoi l'acide nucléique actif est exprimé ou est poussé à être exprimé dans lesdites cellules.

11. Composition pharmaceutique selon la revendication 10, **caractérisée en ce que** les ultrasons sont émis à partir d'un transducteur unique sous forme de faisceau à colonnes, à partir de plus d'un transducteur fournissant une configuration de faisceau d'intersection ou à partir d'un traducteur focalisé.

12. Composition pharmaceutique selon la revendication 10 ou 11, **caractérisée en ce que** les ultrasons sont utilisés à une fréquence de 15 kHz à 10 MHz, en particulier de 15 kHz à 5 MHz.

13. Composition pharmaceutique selon l'une quelconque des revendications 10 à 12, **caractérisée en ce que** la composition est administrée au patient après quoi une étape physique de conversion du précurseur de matrice soluble en une matrice est réalisée.
